# EUROPÄISCHE PATENTSCHRIFT

(11) **EP 1 000 135 B1**
(45) Veröffentlichungstag und Bekanntmachung des Hinweises auf die Patenterteilung: **12.11.2003**
(21) Anmeldenummer: 98943764.5
(22) Anmeldetag: 21.07.1998
(51) Int. Cl.: C11D 3/20, A61K 7/50, C11D 1/94, C11D 1/83, C11D 1/825

(54) **WÄSSRIGE PERLGLANZKONZENTRATE**
AQUEOUS PEARLESCENT CONCENTRATES
CONCENTRES DE LUSTRE PERLAIRE AQUEUX

(30) Priorität: 30.07.1997 DE 19732709
(43) Veröffentlichungstag der Anmeldung: 17.05.2000
(73) Patentinhaber: Cognis Deutschland GmbH & Co. KG, 40589 Düsseldorf (DE)
(72) Erfinder: ANSMANN, Achim, D-40699 Erkrath (DE); KAWA, Rolf, D-40789 Monheim (DE); STRAUSS, Gabriele, D-40589 Düsseldorf (DE); LÖHL, Thorsten, D-40597 Düsseldorf (DE)
(86) Internationale Anmeldenummer: EP9804579
(87) Internationale Veröffentlichungsnummer: WO99006511

(56) Entgegenhaltungen:
- EP-A- 0 569 843
- DE-A- 2 603 803
- DE-A- 19 511 571
- DE-A- 19 622 968
- US-A- 3 489 690

## Beschreibung

### Gebiet der Erfindung

Die Erfindung betrifft wäßrige Perlglanzkonzentrate mit einem Gehalt an Fettketonen, Ethersulfaten und Emulgatoren, ein Verfahren zu ihrer Herstellung, ein weiteres Verfahren zur Herstellung von perlglänzenden oberflächenaktiven Zubereitungen unter Verwendung der Konzentrate sowie die Verwendung der Mischung als Perlglanzkonzentrate.

### Stand der Technik

Der weich schimmemde Glanz von Perlen hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Periglanzwachse, insbesondere vom Typ der Glycolmono- und -difettsäureester von Bedeutung, die überwiegend zur Erzeugung von Perlglanz in Haarshampoos und Duschgelen eingesetzt werden.
Eine Übersicht zu modernen, periglänzenden Formulierungen findet sich von A.Ansmann und R.Kawa in **Parf.Kosm. 75, 578 (1994).**

Der Stand der Technik kennt eine Vielzahl von Formulierungen, die oberflächenaktiven Mitteln den gewünschten Perlglanz verleihen. So sind beispielsweise aus den beiden Deutschen Patentanmeldungen **DE-A1 3843572** und **DE-A1 4103551** (Henkel) Perlglanzkonzentrate in Form fließfähiger wäßriger Dispersionen bekannt, die 15 bis 40 Gew.-% perlglänzender Komponenten, 5 bis 55 Gew.-% Emulgatoren und 0,1 bis 5 bzw. 15 bis 40 Gew.-% Polyole enthalten. Bei den Perlglanzwachsen handelt es sich um acylierte Polyalkylenglycole, Monoalkanolamide, lineare, gesättigte Fettsäuren oder Ketosulfone. In den beiden Europäischen Patentschriften **EP-B1 0181773** und **EP-B1 0285389** (Procter & Gamble) werden Shampoozusammensetzungen vorgeschlagen, die Tenside, nicht-flüchtige Silicone und Perlglanzwachse enthalten. Gegenstand der Europäischen Patentanmeldung **EP-A2 0205922** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP-B1 0569843** (Hoechst) lassen sich nichtionische, fließfähige Perlglanzdispersionen auch erhalten, indem man Mischungen von 5 bis 30 Gew.-% acylierten Polyglycolen und 0,1 bis 20 Gew.-% ausgewählten nichtionischen Tensiden herstellt. Aus der Europäischen Patentanmeldung **EP-A2 0581193** (Hoechst) sind ferner fließfähige, konservierungsmittelfreie, Perlglanzdispersionen bekannt, die acylierte Polyglycolether, Betaine, Aniontenside und Glycerin enthalten. Schließlich wird in der Europäischen Patentanmeldung **EP-A1 0684302** (Th.Goldschmidt) die Verwendung von Polyglycerinestem als Kristallisationshilfsmittel für die Herstellung von Perlglanzkonzentraten vorgeschlagen. Aus der **DE-A1-196 22 968** (Henkel) sind wäßrige Perlglanzkonzentrate bekannt, die Fettketone und Emulgatoren, gegebenenfalls in Abmischung mit Alkylsulfaten enthalten. Die **DE-A1-196 22 968** offenbart aber keine Konzentrate, die ethoxylierte Alkylsulfate enthalten. Gegenstand der DE-A-19511571 sind Perlglanzkonzentrate enthaltend 10-40% perlglanzbildende Komponenten und 15-55% Emulgatoren.

Trotz der Vielzahl von Mitteln besteht im Markt ein ständiges Bedürfnis nach neuen Perlglanzwachsen, die beispielsweise im Gegensatz zu acylierten Polyglycolen keine Ethylenoxideinheiten aufweisen und sich gegenüber den Produkten des Stands der Technik auch bei verminderter Einsatzmenge durch einen brillanten Glanz auszeichnen, die die Mitverwendung kritischer Inhaltsstoffe wie beispielsweise von Siliconen zulassen, ohne daß die Stabilität der Formulierungen beeinträchtigt wird, gleichzeitig über leicht abspaltbare Gruppen verfügen, damit eine ausreichende biologische Abbaubarkeit gewährleistet ist und die insbesondere in konzentrierter Form noch leicht beweglich und damit handhabbar sind. Die Aufgabe der vorliegenden Erfindung hat somit darin bestanden, neue Perlglanzkonzentrate mit dem geschilderten komplexen Anforderungsprofil zur Verfügung zu stellen.

### Beschreibung der Erfindung

Gegenstand der Erfindung sind wäßrige Periglanzkonzentrate, enthaltend
bezogen auf den nicht - wäßrigen Teil der Konzentrate
a) 5 bis 15 Gew.-% Fettketone
b) 20 bis 50 Gew.-% Alkyl- und/oder Alkenylethersulfate der Formel (II) in der R³ für linearen oder

   **R**^{**3**}**O-(CH**_{**2**}**CH**_{**2**}**O)**_{**m**}**SO**_{**3**}**X** **(II)**

   verzweigten Alkyl- und/oder Alkenylrest in 6 bis 22 Kohlenstoffatomen, n für Zahler von 1 bis 10 und x für ein Alkali - und/oder Erdalkalimetall, Ammonium, Alkylammonium, Alkanolammonium oder Glucammonium steht und
c) 1 bis 20 Gew.-% nichtionische, kationische, ampholytische und/oder zwitterionische Emulgatoren

Überraschenderweise wurde gefunden, daß Fettketone in Kombination mit Ethersulfaten ausgezeichnete perlglänzende Eigenschaften besitzen und sich gegenüber den Produkten des Stands der Technik durch eine höhere Brillanz bei geringerer Einsatzmenge, besondere Feinteiligkeit und Lagerstabilität auszeichnen. Die Perlglanzkonzentrate sind leicht biologisch abbaubar, in konzentrierter Form dünnflüssig und erlauben auch die Einarbeitung von problematischen inhalts-stoffen wie beispielsweise Siliconen in kosmetische Zubereitungen.

### Fettketone

Fettketone, die als Komponente (a) in Betracht kommen, folgen der Formel **(I)**,

**R**^{**1**}**-CO-R**^{**2**} **(I)**

in der R¹ und R² unabhängig voneinander für gegebenenfalls hydroxysubstituierte Alkyl- und/oder Alkenylreste mit 1 bis 22 Kohlenstoffatomen stehen, mit der Maßgabe, daß sie in Summe mindestens 16, vorzugsweise 18 und insbesondere 20 bis 36 Kohlenstoffatome aufweisen. Die Ketone können nach Verfahrens des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R¹ und R² aber nur um ein Kohlenstoffatom und leiten sich von Fettsäuren mit 8 bis 18 Kohlenstoffatomen ab. Dabei zeichnen sich Caprinon, Lauron und Stearon durch besonders vorteilhafte Perlglanzeigenschaften aus. Die Einsatzmenge der Fettketone bezogen auf den nicht-wäßrigen Teil der Konzentrate kann 1 bis 30, vorzugsweise 5 bis 15 und insbesondere 8 bis 12 Gew.-% betragen.

### Alkylethersulfate

Alkylethersulfate ("Ethersulfate") stellen bekannte anionische Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden. Im Sinne der Erfindung kommen Ethersulfate in Betracht, die der Formel **(II)** folgen,

**R**^{**3**}**O-(CH**_{**2**}**CH**_{**2**}**O)**_{**m**}**SO**_{**3**}**X** **(II)**

in der R³ für einen linearen oder verzweigten Alkyl- und/oder Alkenylrest mit 6 bis 22 Kohlenstoffatomen, m für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze. Die Ethersulfate können in Mengen von 10 bis 60, vorzugsweise 20 bis 50 und insbesondere 30 bis 40 Gew.-% - bezogen auf den nicht-wäßrigen Teil der Konzentrate - eingesetzt werden.

### Alkyl- und/oder Alkenyloligoglykoside

Alkyl- und Alkenyloligoglykoside stellen bekannte nichtionische Tenside dar, die der Formel **(III)** folgen,

**R**^{**4**}**O-[G]**_{**p**} **(III)**

in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0301298** und **WO 90/03977** verwiesen. Die Alkylund/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁴ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3. Die Glucoside können in Mengen von 10 bis 60, vorzugsweise 20 bis 50 und insbesondere 30 bis 40 Gew.-% - bezogen auf den nicht-wäßrigen Teil der Konzentrate - eingesetzt werden.

### Emulgatoren

Die erfindungsgemäßen Perlglanzkonzentrate können als Emulgatoren nichtionogene Tenside aus mindestens einer der folgenden Gruppen enthalten:
(c1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Triglyceride;
(c2) C_{12/18}-Fettsäuremono- und -diester von Anlagerungsprodukten von 1 bis 30 Mol Ethylenoxid an Glycerin;
(c3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(c4) Anlagerungsprodukte von 15 bis 60 Mol Ethylenoxid an Ricinusöl und/oder gehärtetes Ricinusöl;
(c5) Polyol- und insbesondere Polyglycerinester wie z.B. Polyglycerinpolyricinoleat, Polyglycerinpoly-12-hydroxystearat oder Polyglycerindimerate. Ebenfalls geeignet sind Gemische von Verbindungen aus mehreren dieser Substanzklassen;
(c6) Anlagerungsprodukte von 2 bis 15 Mol Ethylenoxid an Ricinusöl und/oder gehärtetes Ricinusöl;
(c7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(c8) Trialkylphosphate sowie Mono-, Di- und/oder Tri-PEG-alkylphosphate;
(c9) Wollwachsalkohole;
(c10) Polysiloxan-Polyalkyl-Polyether-Copolymere bzw. entsprechende Derivate;
(c11) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(c12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Die erfindungsgemäßen Perlglanzkonzentrate können die Emulgatoren - bezogen auf den nicht-wäßrigen Anteil - in Mengen von 1 bis 20, vorzugsweise 5 bis 15 und insbesondere 8 bis 12 Gew.-% enthalten.

### Polyole

Zur Verbesserung des Fließverhaltens können die Konzentrate als weitere Komponente (d) Polyole, enthalten. Hierbei handelt es sich üblicherweise um Stoffe, die 2 bis 15 Kohlenstoffatome und mindestens zwei Hydroxylgruppen besitzen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere- Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die erfindungsgemäßen Perlglanzkonzentrate können die Polyole, vorzugsweise Glycerin, Propylenglycol, Butylenglycol, Hexylenglycol sowie Polyethylenglycole mit einem durchschnittlichen Molekulargewicht im Bereich von 100 bis 1.0000 in Mengen von 0,1 bis 40, vorzugsweise 0,5 bis 15 und insbesondere 1 bis 5 Gew.-% enthalten.

### Herstellverfahren

In einer bevorzugten Ausführungsform, die ebenfalls Gegenstand der Erfindung ist, erfolgt die Herstellung der Perlglanzkonzentrate, indem man eine Mischung aus den Komponenten (a), (b) und (c) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt. Ferner ist es möglich, eine konzentrierte wäßrige (Anion-)Tensidpaste vorzulegen, das Perlglanzwachs in der Wärme einzurühren und die Mischung anschließend mit weiterem Wasser auf die gewünschte Konzentration zu verdünnen oder das Vermischen in Gegenwart polymerer hydrophiler Verdickungsmittel, wie etwa Hydroxypropylcellulosen, Xanthan Gum oder Polymeren vom Carbomer-Typ durchzuführen. Üblicherweise liegt der nicht-wäßrige Anteil der erfindungsgemäßen Konzentrate im Bereich von 20 bis 60 und vorzugsweise 30 bis 50 Gew.-%.

### Gewerbliche Anwendbarkeit

Die erfindungsgemäßen Perlglanzkonzentrate eignen sich zur Einarbeitung in oberflächenaktiven Zubereitungen wie beispielsweise Haarshampoos oder manuellen Geschirrspülmitteln. Ein weiterer Gegenstand der Erfindung betrifft daher ein Verfahren zur Herstellung getrübter und periglänzender flüssiger, wäßriger Zubereitungen wasserlöslicher grenzflächenaktiver Stoffe, bei dem man den klaren wäßrigen Zubereitungen bei 0 bis 40°C die Perlglanzkonzentrate in einer Menge von 0,5 bis 40, vorzugsweise 1 bis 20 Gew.-% der Zubereitung zusetzt und unter Rühren darin verteilt.

### Tenside

Die oberflächenaktiven Zubereitungen, die in der Regel einen nicht-wäßrigen Anteil im Bereich von 1 bis 50 und vorzugsweise 5 bis 35 Gew.-% aufweisen, können nichtionische, anionische, kationische und/oder amphotere bzw. amphotere Tenside enthalten, deren Anteil an den Mitteln üblicherweise bei etwa 50 bis 99 und vorzugsweise 70 bis 90 Gew.-% beträgt. Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologen Verteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124** oder **J.Falbe (ed.), "Katalysatoren, Tenside und Mineralöladditive", Thieme Verlag, Stuttgart, 1978, S. 123-217** verwiesen. Die gleichen Tenside können auch unmittelbar zur Herstellung der Perlglanzkonzentrate eingesetzt werden, die anionischen Tenside eignen sich auch als Emulgatoren. In diesem Zusammenhang ist der Einsatz von Alkylethersulfaten als anionische Emulgatoren bevorzugt.

### Hilfs- und Zusatzstoffe

Die oberflächenaktiven Zubereitungen, denen die erfindungsgemäßen Perlglanzkonzentrate zugesetzt werden, können weitere Hilfs- und Zusatzstoffe enthalten, wie beispielsweise Ölkörper, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farbstoffe und Parfümöle .

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-; alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Im Sinne der Erfindung können neben den Fettstoffen auch weitere bekannte **Perlglanzwachse** wie insbesondere Mono- und Difettsäureester von Polyalkylenglycolen, Partial- und Triglyceride sowie Ester von Fettalkoholen mit mehrwertigen Carbonsäuren bzw. Hydroxycarbonsäuren eingesetzt werden. Als **Stabilisatoren** können Metallsalze von Fettsäuren wie z.B. Magnesium-, Aluminium- undloder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder die bereits oben genannten Polyole eingesetzt werden. Als **Konsevierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, lonon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

Ein letzter Gegenstand der Erfindung betrifft schließlich die Verwendung der genannten Fettstoffe als Perlglanzwachse zur Herstellung von oberflächenaktiven Zubereitungen.

## Patentansprüche

1. Wäßrige Perlglanzkonzentrate enthaltend, bezogen auf den nicht-wäßrigen Teil der Konzentrate
a) 5 bis 15 Gew.% Fettketone,
b) 20 bis 50 Gew.% Alkyl- und/oder Alkenylethersulfate der Formel (II)
**R**^{**3**}**O-(CH**_{**2**}**CH**_{**2**}**O)**_{**n**}**SO**_{**3**}**X** **(II)**
in der R³ für einen linearen oder verzweigten Alkyl- und/oder Alkylenrest mit 6 bis 22 Kohlenstoffatomen, n für Zahlen von 1 bis 10 und x für ein Alkali- und/oder Erdalkalimetall, Ammonium, Alkylammonium, Alkanolammonium oder Glucammonium steht und
c) 1 bis 20 Gew.% nichtionische, kationische, ampholytische und/oder zwitterionische Emulgatoren.

2. Perlglanzkonzentrate nach Anspruch 1, **dadurch gekennzeichnet, daß** sie als Komponente (a) Fettketone der Formel (I) enthalten,
**R**^{**1**}**-CO-R**^{**2**} **(I)**
in der R¹ und R² unabhängig voneinander für gegebenenfalls hydroxysubstituierte Alkylund/oder Alkenylreste mit 1 bis 22 Kohlenstoffatomen stehen, mit der Maßgabe, daß sie in Summe mindestens 16 Kohlenstoffatome aufweisen.

3. Perlglanzkonzentrate nach den Ansprüchen 1 und 2, **dadurch gekennzeichnet, daß** sie zusätzlich Alkyl- und/oder Alkenyloligoglykoside der Formel (III) enthalten,
**R**^{**4**}**O-[G]**_{**p**} **(III)**
in der R⁴ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

4. Perlglanzkonzentrate nach den Ansprüchen 1 bis 3, **dadurch gekennzeichnet, daß** sie als Komponente (c) Emulgatoren enthalten, die ausgewählt sind aus der Gruppe, die gebildet wird von:
(c1) Anlagerungsprodukten von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenolen mit 8 bis 15 C-Atomen in der Alkylgruppe und an Triglyceride;
(c2) C_{12/18}-Fettsäuremono- und -diester von Anlagerungsprodukten von 1 bis 30 Mol Ethylenoxid an Glycerin;
(c3) Glycerinmono- und -diester und Sorbitanmono- und -diester von gesättigten und ungesättigten Fettsäuren mit 6 bis 22 C-Atomen und deren Ethylenoxidanlagerungsprodukten;
(c4) Anlagerungsprodukte von 15 bis 60 Mol Ethylenoxid and Ricinusöl und/oder gehärtetes Ricinusöl;
(c5) Polyol- und insbesondere Plyglycerinester wie z.B. Polyglycerinpolyricinoleat, Polyglycerinpoly-12-hydroxystearat oder Polyglycerindimerate. Ebenfalls geeignet sind Gemische von Verbindungen aus mehreren dieser Substanzklassen;
(c6) Anlagerungsprodukte von 2 bis 15 Mol Ethylenoxid and Ricinusöl und/oder gehärtetes Ricinusöl;
(c7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Methylglucosid, sowie Polyglucoside (z.B. Cellulose);
(c8) Trialkylphosphate sowie Mono-, Di- und/oder Tri-PEG-alkylphosphate;
(c9) Wollwachsalkohole;
(c10) Polysiloxan-Polyalkyl-Polyether-Copolymere bzw. entsprechende Derivate;
(c11) Mischester, die Kondensationsprodukte aus einem Pentaerythrit-di-fettsäureester und einem Citronensäure-die-fettalkoholester im Molverhältnis 1:1 darstellen, wobei die Veresterung der Citronensäure unter Verwendung von wäßriger Citronensäure erfolgt und/oder Mischester von Fettsäuren mit 6 bis 22 Kohlenstoffatomen, Methylglucose und Polyolen, vorzugsweise Glycerin sowie
(c12) Polyalkylenglycole.

5. Perlglanzkonzentrate nach den Ansprüchen 1 bis 4, **dadurch gekennzeichnet, daß** sie als Komponente (c) Emulgatoren enthalten, bei denen es sich um Anlagerungsprodukte von 2 bis 30 Mol Ethylenoxid und/oder 0 bis 5 Mol Propylenoxid an lineare Fettalkohole mit 8 bis 22 C-Atomen handelt.

6. Perlglanzkonzentrate nach den Ansprüchen 1 bis 5, **dadurch gekennzeichnet, daß** sie als zusätzliche Komponente (d) Polyole enthalten.

7. Verfahren zur Herstellung von Perlglanzkonzentraten nach Anspruch 1, **dadurch gekennzeichnet, daß** man eine Mischung aus den Komponenten (a), (b) und (c) herstellt, auf eine Temperatur erwärmt, die 1 bis 30 °C oberhalb des Schmelzpunktes der Mischung liegt, mit der erforderlichen Menge Wasser etwa der gleichen Temperatur mischt und anschließend auf Raumtemperatur abkühlt.

8. Verfahren zur Herstellung getrübter und perlglänzender flüssiger, wäßriger Zubereitungen wasserlöslicher grenzflächenaktiver Stoffe, bei dem man den klären wäßrigen Zubereitungen bei 0 bis 40 °C Perlglanzkonzentrate nach den Ansprüchen 1 bis 6 in einer Menge von 0,5 bis 40 Gew.% der Zubereitung zusetzt und unter Rühren darin verteilt.

9. Verwendung von Mischungen nach den Ansprüchen 1 bis 6 als Perlglanzkonzentrate zur Herstellung von oberflächenaktiven Zubereitungen.

## Claims

1. Aqueous pearlizing concentrates containing - based on the nonaqueous part of the concentrates -
(a) 5 to 15% by weight of fatty ketones,
(b) 20 to 50% by weight of alkyl and/or alkenyl ether sulfates corresponding to formula **(II)**:
**R**^{**3**}**O-(CH**_{**2**}**CH**_{**2**}**O)**_{**n**}**SO**_{**3**}**X** **(II)**
in which R³ is a linear or branched alkyl and/or alkenyl group containing 6 to 22 carbon atoms, n is a number of 1 to 10 and X is an alkali metal and/or alkaline earth metal, ammonium, alkylammonium, alkanolammonium or glucammonium and
(c) 1 to 20% by weight of nonionic, cationic, ampholytic and/or zwitterionic emulsifiers.

2. Pearlizing concentrates as claimed in claim 1, **characterized in that** they contain as component (a) fatty ketones corresponding to formula **(I)**:
**R**^{**1**}**-CO-R**^{**2**} **(I)**
in which R¹ and R² independently of one another represent optionally hydroxysubstituted alkyl and/or alkenyl groups containing 1 to 22 carbon atoms, with the proviso that, in all, they contain at least 16 carbon atoms.

3. Pearlizing concentrates as claimed in claims 1 and 2, **characterized in that** they additionally contain alkyl and/or alkenyl oligoglycosides corresponding to formula **(III)**:
**R**^{**4**}**O-[G]**_{**p**} **(III)**
where R⁴ is an alkyl and/or alkenyl radical containing 4 to 22 carbon atoms, G is a sugar unit containing 5 or 6 carbon atoms and p is a number of 1 to 10.

4. Pearlizing concentrates as claimed in claims 1 to 3, **characterized in that** they contain as component (c) emulsifiers selected from the group consisting of:
(c1) addition products of 2 to 30 mol ethylene oxide and/or 0 to 5 mol propylene oxide onto linear fatty alcohols containing 8 to 22 carbon atoms, onto fatty acids containing 12 to 22 carbon atoms, onto alkylphenols containing 8 to 15 carbon atoms in the alkyl group and onto triglycerides;
(c2) C_{12/18} fatty acid monoesters and diesters of addition products of 1 to 30 mol ethylene oxide onto glycerol;
(c3) glycerol monoesters and diesters and sorbitan monoesters and diesters of saturated and unsaturated fatty acids containing 6 to 22 carbon atoms and ethylene oxide adducts thereof;
(c4) addition products of 15 to 60 mol ethylene oxide onto castor oil and/or hydrogenated castor oil;
(c5) polyol esters and, in particular, polyglycerol esters such as, for example, polyglycerol polyricinoleate, polyglycerol poly-12-hydroxy-stearate or polyglycerol dimerates. Mixtures of compounds from several of these classes are also suitable;
(c6) addition proucts of 2 to 15 mol ethylene oxide onto castor oil and/or hydrogenated castor oil;
(c7) partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), methyl glucoside and polyglucosides (for example cellulose);
(c8) trialkyl phosphates and mono-, di- and/or tri-PEG-alkyl phosphates;
(c9) wool wax alcohols;
(c10) polysiloxane/polyalkyl polyether copolymers and corresponding derivatives;
(c11) mixed esters in the form of condensation products of a pentaerythritol difatty acid ester and a citric acid difatty alcohol ester in a molar ratio of 1:1, the citric acid being esterified using aqueous citric acid, and/or mixed esters of fatty acids containing 6 to 22 carbon atoms, methyl glucose and polyols, preferably glycerol, and
(c12) polyalkylene glycols.

5. Pearlizing concentrates as claimed in claims 1 to 4, **characterized in that** they contain as component (c) emulsifiers in the form of addition products of 2 to 30 mol ethylene oxide and/or 0 to 5 mol propylene oxide onto linear fatty alcohols containing 8 to 22 carbon atoms.

6. Pearlizing concentrates as claimed in claims 1 to 5, **characterized in that** they contain polyols as an additional component (d).

7. A process for the production of the pearlizing concentrates claimed in claim 1, **characterized in that** a mixture of components (a), (b) and (c) is prepared, heated to a temperature 1 to 30°C above the melting point of the mixture, mixed with the necessary quantity of water having substantially the same temperature and then cooled to room temperature.

8. A process for the production of opaque and pearlescent liquid water-containing preparations of water-soluble surface-active substances, in which the pearlizing concentrates claimed in claims 1 to 6 are added to the clear aqueous preparations at 0 to 40°C in a quantity of 0.5 to 40% by weight, based on the preparation, and distributed therein by stirring.

9. The use of the mixtures claimed in claims 1 to 6 as pearlizing concentrates for the production of surface-active formulations.

## Revendications

1. Concentrés à éclat nacré aqueux, contenant rapporté à la partie non aqueuse des concentrés,
a) 5 à 15 % en poids de cétones grasses,
b) 20 à 50 % en poids de sulfate d'éther d'alkyle et/ou d'alkényle de formule (II)
R³O-(CH₂CH₂O)ₙSO₃X (II)
dans laquelle R³ représente un reste alkyl et/ou alkylène linéaire ou ramifié ayant de 6 à 22 atomes de carbone, n représente des nombres allant de 1 à 10, et X représente un métal alcalin et/ou alcalinoterreux, un ammonium, un alkylammonium, un alkanolammonium, ou un glucammonium, et
c) 1 à 20 % en poids d'agents émulsionnants non ioniques, cationiques, ampholytiques et/ou zwitterioniques.

2. Concentrés à éclat nacré selon la revendication 1,
**caractérisés en ce qu'**
ils renferment comme composant a) des cétones grasses de formule (I)
R¹-CO-R² (I)
dans laquelle R¹ et R² indépendamment l'un de l'autre, représentent des restes alkyle et/ou alkényle ayant de 1 à 22 atomes de carbone éventuellement substitués par un hydroxy, avec la précision que globalement ils possèdent au moins 16 atomes de carbone.

3. Concentrés à éclat nacré selon la revendication 1 ou 2,
**caractérisés en ce qu'**
ils renferment en supplément des alkyl et/ou alkényloligoglycosides de formule (III)
R⁴O-[G]ₚ (III)
dans laquelle R⁴ représente un reste alkyle et/ou alkényle ayant de 4 à 22 atomes de carbone, G représente un reste de sucre ayant 5 ou 6 atomes de carbone et p représente des nombres allant de 1 à 10.

4. Concentrés à éclat nacré selon les revendications 1 à 3,
**caractérisés en ce qu'**
ils renferment comme composant c) des agents émulsionnants qui sont choisis dans le groupe formé :
(c1) des produits d'addition de 2 à 30 mols d'oxyde d'éthylène et/ou de 0 à 5 mois d'oxyde de propylène sur des alcools gras linéaires ayant de 8 à 22 atomes de carbone, sur des acides gras ayant de 12 à 22 atomes de carbone, sur des alkylphénols ayant de 8 à 15 atomes de carbone dans le groupe alkyle et sur des triglycérides,
(c2) des mono- et des diesters d'acide gras en C₁₂-C₁₈ de produits d'addition de 1 à 30 mois d'oxyde d'éthylène sur le glycérol,
(c3) des mono- et des diesters de glycérol et des mono- et des diesters de sorbitane d'acides gras saturés et non saturés ayant de 6 à 22 atomes de carbone et leurs produits d'addition de l'oxyde d'éthylène,
(c4) des produits d'addition de 15 à 60 mois d'oxyde d'éthylène et d'huile de ricin et/ou d'huile de ricin durcie,
(c5) des esters de polyol et en particulier de polyglycérol comme par exemple un polyricinoléate de polyglycérol, un poly-12-hydroxystéarate de polyglycérol ou un dimère de polyglycérol, conviennent également des mélanges de composés issus de plusieurs de ces classes de substances,
(c6) des produits d'addition de 2 à 15 mols d'oxyde d'éthylène et d'huile de ricin et/ou d'huile de ricin durcie,
(c7) des esters partiels à base d'acides gras linéaires ramifiés, non saturés ou saturés, ayant de C₆ à C₂₂ atomes de carbone, d'acide ricinoléique, ainsi que d'acide 12-hydroxystéarique et de glycérol ; de polyglycérol, de pentaérythritol, de di-pentaérythritol, d'alcools de sucre, par exemple le sorbitol, le méthylglucoside ainsi que des polyglucosides (par exemple la cellulose),
(c8) des phosphates de trialkyle ainsi que des mono-, des di- et/ou des tri PEG phosphates d'alkyle,
(c9) des alcools de cire de laine,
(c10) des copolymères de polysiloxane -polyalkyle - polyéther ou les dérivés correspondants,
(c11) des esters mixtes qui représentent des produits de condensation à partir de diester d'acide gras de pentaérythritol et d'un di-ester d'alcool gras d'acide citrique dans un rapport molaire de 1 :1, dans lesquels l'estérification de l'acide citrique s'effectue en utilisant de l'acide citrique aqueux et/ou des esters mixtes d'acide gras ayant de 6 à 22 atomes de carbone, du méthylglucose et des polyols, de préférence du glycérol ainsi que
(c12) des polyalkylèneglycols.

5. Concentrés à éclat nacré selon les revendications 1 à 4,
**caractérisés en ce qu'**
ils renferment comme composant (c) des agents émulsionnants pour lesquels il s'agit de produits d'addition de 2 à 30 mois d'oxyde d'éthylène et/ou de 0 à 5 mols d'oxyde de propylène sur des alcools gras linéaires ayant de 8 à 22 atomes de carbone.

6. Concentrés à éclat nacré selon les revendications 1 à 5,
**caractérisés en ce qu'**
ils renferment comme composants (d) supplémentaires des polyols.

7. Procédé de production de concentrés à éclat nacré selon la revendication 1,
**caractérisé en ce qu'**
on prépare un mélange à base des composants (a), (b) et (c), on chauffe à une température qui se situe de 1 à 30°C au-dessus du point de fusion du mélange, on mélange avec la quantité requise d'eau d'une température à peu près identique et ensuite on refroidit à la température ambiante.

8. Procédé de préparations aqueuses opalescentes et liquides à éclat nacré, de substances solubles dans l'eau activés sur la tension superficielle, selon lequel à des préparations aqueuses limpides, à 0 à 40°C, on ajoute des concentrés à éclat nacré selon les revendications 1 à 6, en une quantité allant de 0,5 à 40 % en poids de la préparation et on les répartit sous agitation dans celle-ci.

9. Utilisation de mélanges selon les revendications 1 à 6,
comme concentrés à éclat nacré en vue de la production de préparations actives sur la tension superficielle.
